# EUROPEAN PATENT SPECIFICATION

(11) **EP 4 337 632 B1**
(45) Date of publication and mention of the grant of the patent: **26.11.2025**
(21) Application number: 22724858.0
(22) Date of filing: 11.05.2022
(51) Int. Cl.: A61P 25/08, C07C 69/157, C07C 39/42

(54) **RESORCINOL DERIVATIVE AS A PHARMACEUTICALLY ACTIVE COMPOUND AND METHOD OF PREPARATION THEREOF**
RESORCINOLDERIVAT ALS PHARMAZEUTISCH AKTIVE VERBINDUNG UND VERFAHREN ZU DESSEN HERSTELLUNG
DÉRIVÉ DE RÉSORCINOL UTILISÉ EN TANT QUE COMPOSÉ PHARMACEUTIQUEMENT ACTIF ET SON PROCÉDÉ DE PRÉPARATION

(30) Priority: 12.05.2021 GB 202106786
(43) Date of publication of application: 20.03.2024
(73) Proprietor: Jazz Pharmaceuticals Research UK Limited, Sittingbourne Kent ME9 8AG (GB)
(72) Inventor: SILCOCK, Alan James, Sittingbourne, Kent ME9 8AG (GB); TSE, Karen Ka-Yen, Sittingbourne, Kent ME9 8AG (GB); OSBORNE, James Daniel, Sittingbourne, Kent ME9 8AG (GB); HINCHLIFFE, Paul Stuart, Saffron Walden CB10 1XL (GB); SHARPE, Andrew, Saffron Walden CB10 1XL (GB)
(74) Representative: HGF
(86) International application number: PCT/GB2022/051200
(87) International publication number: WO 2022/238701

(56) References cited:
- WO-A1-2020/232545
- WO-A1-2021/099783
- WO-A1-99/53917
- CN-A- 112 592 260
- TONY K.M. SHING ET AL: "Mild Manganese(III) Acetate Catalyzed Allylic Oxidation: Application to Simple and Complex Alkenes", ORGANIC LETTERS, vol. 8, no. 14, 1 January 2006 (2006-01-01), pages 3149 - 3151, XP055578979
- BAILEY SOPHIA J. ET AL: "Lewis-Acid-Mediated Union of Epoxy-Carvone Diastereomers with Anisole Derivatives: Mechanistic Insight and Application to the Synthesis of Non-natural CBD Analogues", ORGANIC LETTERS, vol. 20, no. 15, 23 July 2018 (2018-07-23), US, pages 4618 - 4621, XP055941238, ISSN: 1523-7060, DOI: 10.1021/acs.orglett.8b01909

## Description

### RELATED APPLICATIONS

The present application is related to, and claims the benefit of, GB 2106786.3 filed on 12 May 2021 (12.05.2021).

### FIELD OF THE INVENTION

The present invention relates to a resorcinol derivative as a pharmaceutically active compound, a research tool, and to a method of preparation thereof.

### BACKGROUND TO THE INVENTION

Resorcinol has been widely known as a versatile chemical compound used extensively in the development of advanced chemistries and technologies (Durairaj, 2005). It has been employed in a wide range of applications from medical to chemical, including in the manufacture of resins, plastics, dyes, medicine, and numerous other organic chemical compounds.

Resorcinol appears as white crystals or powder and has a faint, characteristic aromatic odour, with a sweetish bitter taste, and is readily soluble in water and alcohol. Other names for the compound include resorcin, meta-dihydroxybenzene, 1,3-dihdroxybenzene, 1,3-benzenediol and 3-hydroxyphenol, and its empirical formula is C₆H₆O₂. Structurally, the compound has two hydroxyl groups in the aromatic ring structure, which are located at the meta-positions with respect to each hydroxyl group. The high reactivity of resorcinol is primarily associated with the location of these two hydroxyl groups in the benzene ring, with the hydrogen atoms adjacent to the hydroxyl groups being particularly reactive.

Production of resorcinol can be via several routes, either by using a natural resin such as a distillate of brazilwood, melting any of a large number of resins (such as galbanum and asafoetida) and combining it with potassium hydroxide, or by several synthetic methods. One synthetic manufacturing method is sulfonation of benzene with sulfuric acid and fusing the resulting benzenedisulfonic acid with sodium hydroxide, and subsequently extracting the resorcinol.

One of the uses of resorcinol is its role as a chemical intermediate for the synthesis of pharmaceuticals and other organic compounds. For example, it is used in the production of diazo dyes and plasticizers and as a UV absorber in resins. As a medicine, resorcinol is used as an antiseptic and disinfectant in topical pharmaceutical products in the treatment of skin disorders and infections such as acne, seborrheic dermatitis, eczema, psoriasis, corns, calluses, and warts. Resorcinol works by helping to remove hard, scaly, or roughened skin as it exerts a keratolytic activity. However, the main use of resorcinol is in the production of resins. Reaction with formaldehyde produces resins used to make rayon and nylon amenable to impregnation with rubber, and as adhesives.

International patent application, WO1999/053917 discloses certain cannabinoids useful as neuroprotective antioxidants.

### BRIEF SUMMARY OF THE INVENTION

At its most general, the present invention relates to a novel resorcinol derivative which is biologically active and hence useful in the treatment of diseases. Such a novel compound may be administered by a wide variety of routes including but not limited to oral, transdermal, buccal, nasal, pulmonary, rectal or ocular. Such compounds may be used for the treatment or prevention of a medical condition including but not limited to epilepsy.

In a first aspect of the invention there is provided a compound of formula (I), or a salt or stereoisomer thereof:

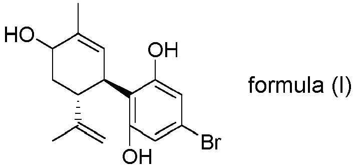

In one embodiment, the compound of formula (I) is (1'R,2'R,4'R)-4-bromo-5'-methyl-2'-(prop-1-en-2-yl)-1',2',3',4'-tetrahydro-[1,1'-biphenyl]-2,4',6-triol, or a salt thereof.

In one embodiment, the compound of formula (I) is (1'R,2'R,4'S)-4-bromo-5'-methyl-2'-(prop-1-en-2-yl)-1',2',3',4'-tetrahydro-[1,1'-biphenyl]-2,4',6-triol, or a salt thereof.

In a second aspect of the invention there is provided a pharmaceutical composition comprising the compound of the first aspect together with one or more additional ingredients selected from carriers, diluents, excipients, adjuvants, fillers, buffers, binders, disintegrants, preservatives, antioxidants, lubricants, stabilisers, solubilisers, surfactants (e.g., wetting agents), masking agents, colouring agents, flavouring agents, and sweetening agents.

Preferably the pharmaceutical composition of the second aspect is in a form selected from a liquid, a solution, a suspension, an emulsion, a syrup, an electuary, a mouthwash, a drop, a tablet, a granule, a powder, a lozenge, a pastille, a capsule, a cachet, a pill, an ampoule, a bolus, a suppository, a pessary, a tincture, a gel, a paste, an ointment, a cream, a lotion, an oil, a foam, a spray, and an aerosol.

In a third aspect of the invention there is provided the compound of the first aspect, or a pharmaceutical composition of the second aspect, for use in a method of treatment.

Preferably, the method of treatment of the third aspect is a method of treatment of epilepsy, generalised seizure, focal onset seizure or tonic-clonic seizure.

In a fourth aspect of the invention there is provided a compound of the first aspect, or a pharmaceutical composition of the second aspect, for use as a medicament.

Preferably, the medicament of the fourth aspect is a medicament for treating epilepsy, generalised seizure, focal onset seizure or tonic-clonic seizure.

Disclosed herein, but not forming part of the present invention, is a method of treatment comprising administering to a subject in need of treatment a therapeutically effective amount of the compound of the first aspect or the pharmaceutical composition of the second aspect.

In accordance with a fifth aspect of the present invention there is provided a process for the production of a compound of formula (I) comprising the following steps:
i) Reacting 5-bromobenzene-1,3-diol with 4-isopropenyl-1-methyl-cyclohex-2-en-1-ol;
ii) Treating the resulting compound 5-Bromo-2-[6-isopropenyl-3-methyl-cyclohex-2-en-1-yl]benzene-1,3-diol with acetic anhydride;
iii) Treating the further resulting compound [3-Acetoxy-5-bromo-2-[6-isopropenyl-3-methyl-cyclohex-2-en-1-yl]phenyl] acetate with manganese acetate dihydrate;
iv) Adding sodium borohydride to the subsequently further resulting compound [3-Acetoxy-5-bromo-2-[(1R,6R)-6-isopropenyl-3-methyl-4-oxo-cyclohex-2-en-1-yl]phenyl] acetate, followed by subsequent steps to produce the compound of Formula I.

These and other aspects and embodiments of the invention are described in further detail below.

### BRIEF SUMMARY OF THE DRAWINGS

The present invention is described with reference to the figures listed below:
**Figure 1** shows the evaluation of the test compound in the mini-MEST test in the mouse as described in Example 2.
**Figure 2** shows the evaluation of the test compound in the MEST test in the mouse as described in Example 3.
**Figure 3** shows the percentage (%) of mice with wild running when administered Compound I in the audiogenic seizure test in the mouse (positive control: valproate; negative control: vehicle) as described in Example 4.
**Figure 4** shows the percentage (%) of mice with clonic convulsion when administered Compound I in the audiogenic seizure test in the mouse (positive control: valproate; negative control: vehicle) as described in Example 4.
**Figure 5** shows the latency to clonic convulsions when administered Compound I in the audiogenic seizure test in the mouse (positive control: valproate; negative control: vehicle) as described in Example 4.
**Figure 6** shows the percentage (%) of mice with tonic extension when administered Compound I in the audiogenic seizure test in the mouse (positive control: valproate; negative control: vehicle) as described in Example 4.
**Figure 7** shows the latency to tonic extension when administered Compound I in the audiogenic seizure test in the mouse (positive control: valproate; negative control: vehicle) as described in Example 4.
**Figure 8** shows the percentage (%) of death when administered Compound I in the audiogenic seizure test in the mouse (positive control: valproate; negative control: vehicle) as described in Example 4.
**Figure 9** shows the evaluation of the test compound in the 6Hz psychomotor test in the mouse as described in Example 5.

### DETAILED DESCRIPTION OF THE INVENTION

The present invention provides a novel resorcinol derivative which is biologically active and hence useful in the treatment of diseases.

### Novel Resorcinol

The invention provides a compound of formula (I):

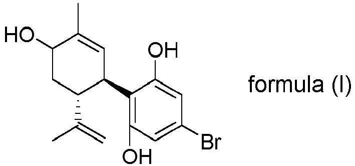

The compound of formula (I) exists as isomers (epimers). The two isomers of the novel resorcinol of the invention are (1'R,2'R,4'R)-4-bromo-5'-methyl-2'-(prop-1-en-2-yl)-1',2',3',4'-tetrahydro-[1,1'-biphenyl]-2,4',6-triol, which will be referred to as Isomer 1, and (1'R,2'R,4'S)-4-bromo-5'-methyl-2'-(prop-1-en-2-yl)-1',2',3',4'-tetrahydro-[1,1'-biphenyl]-2,4',6-triol, which will be referred to as Isomer 2. Isomers 1 and 2 will be referred to collectively as the compound of Formula I or Compound I.

In one embodiment, the compound of formula (I) is (1'R,2'R,4'R)-4-bromo-5'-methyl-2'-(prop-1-en-2-yl)-1',2',3',4'-tetrahydro-[1,1'-biphenyl]-2,4',6-triol (isomer 1).

In one embodiment, the compound of formula (I) is (1'R,2'R,4'S)-4-bromo-5'-methyl-2'-(prop-1-en-2-yl)-1',2',3',4'-tetrahydro-[1,1'-biphenyl]-2,4',6-triol (isomer 2).

### Salts

In some embodiments, the compound of formula (I) is provided in free base form.

Alternatively, it may be convenient or desirable to prepare, purify, and/or handle a corresponding salt of the compound, for example, a pharmaceutically-acceptable salt. Examples of pharmaceutically acceptable salts are discussed in "Pharmaceutical Salts: Properties, Selection, and Use", 2nd Edition, 2002, Stahl and Wermuth (Eds), Wiley-VCH, Weinheim, Germany.

Accordingly, in some embodiments the compound of formula (I) is provided as a salt, for example in a protonated form together with a suitable counter anion.

Suitable counter anions include both organic and inorganic anions. Example of suitable inorganic anions include those derived from inorganic acids, including chloride (Cl⁻), bromide (Br), iodide (I⁻), sulfate (SO₄²⁻), sulfite (SO₃²⁻), nitrate (NO₃⁻), nitrite (NO₂⁻), phosphate (PO₄³⁻), and phosphite (PO₃³⁻). Examples of suitable organic anions include 2-acetoxybenzoate, acetate, ascorbate, aspartate, benzoate, camphorsulfonate, cinnamate, citrate, edetate, ethanedisulfonate, ethanesulfonate, formate, fumarate, gluconate, glutamate, glycolate, hydroxymalate, carboxylate, lactate, laurate, maleate, malate, methanesulfonate, oleate, oxalate, palmitate, phenylacetate, phenylsulfonate, propionate, pyruvate, salicylate, stearate, succinate, sulfanilate, tartarate, toluenesulfonate, and valerate. Examples of suitable polymeric organic anions include those derived from tannic acid and carboxymethyl cellulose.

Alternatively, in some embodiments the compound of formula (I) is provided as a salt, for example in a deprotonated form together with a suitable counter cation.

Suitable counter cations include both organic and inorganic cations. Examples of suitable inorganic cations include alkali metal ions such as Na⁺ and K⁺, alkaline earth cations such as Ca²⁺ and Mg²⁺, and other cations such as Al³⁺. Examples of suitable organic cations include the ammonium ion (i.e., NH₄⁺) and substituted ammonium ions (e.g., NH₃R⁺, NH₂R₂⁺, NHR₃⁺, NR₄⁺). Examples of substituted ammonium ions include those derived from ethylamine, diethylamine, dicyclohexylamine, triethylamine, butylamine, ethylenediamine, ethanolamine, diethanolamine, piperazine, benzylamine, phenylbenzylamine, choline, meglumine, and tromethamine, as well as amino acids, such as lysine and arginine. An example of a common quaternary ammonium ion is N(CH₃)₄⁺.

### Solvates

In some embodiments, the compound of formula (I) is provided in desolvated form, for example, in dehydrated form.

Alternatively, it may be convenient or desirable to prepare, purify, and/or handle a corresponding solvate of the compound.

Accordingly, in some embodiments the compound of formula (I) is provided in the form of a solvate (a complex of solute (e.g., compound, salt of compound) and solvent). Examples of solvates include hydrates, for example, a mono-hydrate, a di-hydrate and a trihydrate.

### Methods of Synthesis

The invention also provides a process for the production of a compound of formula (I) comprising the following steps:
**i)** Reacting 5-bromobenzene-1,3-diol with 4-isopropenyl-1-methyl-cyclohex-2-en-1-ol;
**ii)** Treating the resulting compound 5-Bromo-2-[6-isopropenyl-3-methyl-cyclohex-2-en-1-yl]benzene-1,3-diol with acetic anhydride;
**iii)** Treating the further resulting compound [3-Acetoxy-5-bromo-2-[6-isopropenyl-3-methyl-cyclohex-2-en-1-yl]phenyl] acetate with manganese acetate dihydrate;
**iv)** Adding sodium borohydride to the subsequently further resulting compound [3-Acetoxy-5-bromo-2-[(1R,6R)-6-isopropenyl-3-methyl-4-oxo-cyclohex-2-en-1-yl]phenyl] acetate, followed by subsequent steps to produce the compound of formula (I).

### Intermediates

The invention also provides an intermediate formed in the process of the production of a compound of formula (I), wherein the intermediate is:

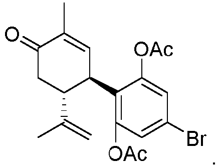

### Pharmaceutical Compositions

While it is possible for the compound of formula (I) to be administered alone, it is preferable to administer a pharmaceutical composition (e.g., a formulation, preparation, or medicament) comprising the compound of formula (I) together with one or more other pharmaceutically acceptable ingredients.

Accordingly, the invention provides a pharmaceutical composition comprising a compound of formula (I), or a salt thereof, together with one or more pharmaceutically acceptable ingredients.

Suitable pharmaceutically acceptable ingredients (e.g. carriers, diluents, excipients, etc.) can be found in standard pharmaceutical texts, for example, Remington: The Science and Practice of Pharmacy, 20th Edition, 2000, pub. Lippincott, Williams & Wilkins; and Handbook of Pharmaceutical Excipients, 2nd edition, 1994.

Examples of suitable pharmaceutically acceptable ingredients include pharmaceutically acceptable carriers, diluents (e.g. oils), excipients, adjuvants, fillers, buffers, binders, disintegrants, preservatives, antioxidants, lubricants, stabilisers, solubilisers, surfactants (e.g., wetting agents), masking agents, colouring agents, flavouring agents, and sweetening agents.

In a preferred embodiment, the pharmaceutical composition comprises, one or more of: an excipient selected among a carrier, an oil, a disintegrant, a lubricant, a stabilizer, a flavouring agent, an antioxidant, a diluent and another pharmaceutically effective compound.

The pharmaceutical composition may be in any suitable form. Examples of suitable forms include liquids, solutions (e.g., aqueous, nonaqueous), suspensions (e.g., aqueous, non-aqueous), emulsions (e.g., oil-in-water, water-in-oil), syrups, electuaries, mouthwashes, drops, tablets (including, e.g., coated tablets), granules, powders, lozenges, pastilles, capsules (including, e.g., hard and soft gelatin capsules), cachets, pills, ampoules, boluses, suppositories, pessaries, tinctures, gels, pastes, ointments, creams, lotions, oils, foams, sprays, and aerosols.

In a preferred embodiment, the form of the pharmaceutical composition is selected from a tablet, a capsule, a granule, a powder for inhalation, a sprinkle, an oral solution and a suspension.

### Medical Treatment

The inventors have found that the compound of formula (I) is biologically active. The worked examples demonstrate that the compound of formula (I) displays anticonvulsant activity in a mouse model. As such, the compound of formula (I) and its salts, as well as pharmaceutical compositions comprising the compound of formula (I) or its salts, will be useful in medical treatment.

Accordingly, the invention provides a compound of formula (I), or a salt thereof, for use in a method of treatment, for example for use in a method of treatment of the human or animal body by therapy (i.e. a method of therapy).

The invention also provides a compound of formula (I), or a salt thereof, for use as a medicament.

Disclosed herein, but not forming part of the present invention is provided a method of treatment comprising administering to a subject in need of treatment a therapeutically effective amount of compound (I), or a salt thereof.

The invention also provides the use of compound (I), or a salt thereof, for the manufacture of a medicament.

### Conditions Treated

The inventors have found that the compound of formula (I) displays anticonvulsant activity in a mouse model of generalised seizure. Accordingly, the compound of formula (I), its salts, as well as pharmaceutical compositions comprising the compound of formula (I) or its salts, will be useful in the treatment of certain conditions associated with seizure.

Similarly, the compound of formula (I), its salts, as well as pharmaceutical compositions comprising the compound of formula (I) or its salts, will be useful as medicaments for treating (and in the manufacture of medicaments for treating) certain conditions associated with seizure.

In a preferred embodiment, the condition associated with seizure is epilepsy.

In one embodiment, the condition associated with seizure is generalised seizure, such as generalised seizure associated with epilepsy.

In one embodiment, the condition associated with seizure is tonic-clonic seizures, such as tonic-clonic seizures associated with epilepsy.

### The Subject/Patient

The method of treatment typically comprises administering a compound of formula (I), or a salt thereof, to a subject or patient.

The subject/patient may be a chordate, a vertebrate, a mammal, a placental mammal, a marsupial (e.g., kangaroo, wombat), a rodent (e.g., a guinea pig, a hamster, a rat, a mouse), murine (e.g., a mouse), a lagomorph (e.g., a rabbit), avian (e.g., a bird), canine (e.g., a dog), feline (e.g., a cat), equine (e.g., a horse), porcine (e.g., a pig), ovine (e.g., a sheep), bovine (e.g., a cow), a primate, simian (e.g., a monkey or ape), a monkey (e.g., marmoset, baboon), an ape (e.g., gorilla, chimpanzee, orang-utan, gibbon), or a human. Furthermore, the subject/patient may be any of its forms of development, for example, an infant or child.

In a preferred embodiment, the subject/patient is a human, more preferably an adult human.

The subject/patient may also be a non-human mammal used in laboratory research, such as a rodent. Rodents include rats, mice, guinea pigs and chinchillas.

### Routes of Administration

The method of treatment may comprise administering a compound of formula (I), or a salt thereof, to a subject by any convenient route of administration, whether systemically/peripherally or topically (i.e., at the site of desired action).

The route of administration may be oral (e.g., by ingestion); buccal; sublingual; transdermal (including, e.g., by a patch, plaster, etc.); transmucosal (including, e.g., by a patch, plaster, etc.); intranasal (e.g., by nasal spray); ocular (e.g., by eyedrops); pulmonary (e.g., by inhalation or insufflation therapy using, e.g., via an aerosol, e.g., through the mouth or nose); rectal (e.g., by suppository or enema); vaginal (e.g., by pessary); parenteral, for example, by injection or infusion, including subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, and intrasternal; or by implant of a depot or reservoir, for example, subcutaneously or intramuscularly.

### Dosages

The method of treatment typically comprises administering a therapeutically effective amount of a compound of formula (I), or a salt thereof, to a subject.

Appropriate dosages of the compound of formula (I), its salts, as well as pharmaceutical compositions comprising the compound of formula (I) or its salts, can vary from patient to patient. Determining the optimal dosage will generally involve balancing the level of therapeutic benefit against any risk or deleterious side effects. The selected dosage level will depend on a variety of factors including, but not limited to, the activity of the particular compound of formula (I), the route of administration, the time of administration, the rate of excretion of the compound, the duration of the treatment, other active agents, compounds, and/or materials used in combination, the severity of the condition, and the species, sex, age, weight, condition, general health, and prior medical history of the patient. The dosage and route of administration will ultimately be at the discretion of the clinician, although generally the dosage will be selected to achieve local concentrations at the site of action which achieve the desired effect without causing substantial harmful or deleterious side-effects.

Administration can be effected in one dose, continuously or intermittently (e.g., in divided doses at appropriate intervals) throughout the course of treatment. Single or multiple administrations can be carried out with the dose level and pattern being selected by the treating clinician.

### Other Aspects and Embodiments

Each and every compatible combination of the embodiments described above is explicitly discloses herein, as if each and every combination was individually and explicitly recited.

Various further aspects and embodiment of the present invention will be apparent to those skilled in the art in view of the present disclosure.

Where used, "and/or" is to be taken as a specific disclosure of each of the relevant components or features alone as well as a specific disclosure of the combination of the components or features. For example, "A and/or B" is to be taken as specific disclosure of each of i) A, ii) B, and iii) A and B, just as if each were set out individually.

Unless context dictates otherwise, the descriptions and definitions of the features set out above are not limited to any particular aspect or embodiment of the invention and apply equally to all aspects and embodiments which are described.

### Definitions

The following definitions are provided in order to aid understanding of the invention.

A "resorcinol" is a compound with the following structure or any super-structure containing such a structure within it:

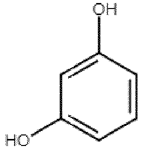

Epilepsy is considered to be a disease of the brain defined by any of the following conditions: (1) At least two unprovoked (or reflex) seizures occurring >24 h apart; (2) one unprovoked (or reflex) seizure and a probability of further seizures similar to the general recurrence risk (at least 60%) after two unprovoked seizures, occurring over the next 10 years; (3) diagnosis of an epilepsy syndrome (A practical clinical definition of epilepsy by the International League Against Epilepsy (ILAE), 2014).

The term "generalized seizure" ("generalized onset seizures") refers to seizures conceptualized as originating at some point within the brain and rapidly engaging bilaterally distributed networks (Operational Classification of Seizure Types by the ILAE, 2017).

A "tonic-clonic seizure" occurs in two phases, a tonic phase typically involving muscle stiffening and loss of consciousness, and a clonic phase typically involving rhythmically jerking of the limbs.

The term "pharmaceutically acceptable" pertains to compounds, ingredients, materials, compositions, dosage forms, etc., which are, within the scope of sound medical judgment, suitable for use in contact with the tissues of the subject in question (e.g., human) without excessive toxicity, irritation, allergic response, or other problem or complication, commensurate with a reasonable benefit/risk ratio. Each ingredient (e.g. carrier, diluent, excipient, etc.) must also be "acceptable" in the sense of being compatible with the other ingredients of the composition.

The term "therapeutically-effective amount" pertains to that amount of a compound, or a material, composition or dosage form comprising a compound, which is effective for producing some desired therapeutic effect, commensurate with a reasonable benefit/risk ratio, when administered in accordance with a desired treatment regimen.

### WORKED EXAMPLES

Certain aspects and embodiments of the invention will not be illustrated by way of example and with reference to the figures described above.

### Example 1: Synthetic Production Method for Resorcinol Derivative

This example describes a novel method of synthesis which was used to produce a novel resorcinol which demonstrated pharmacological activity. Scheme 1 below describes the four stages of the reaction which was used to produce the novel resorcinol, formed via a number of intermediates.

The two isomers of the novel resorcinol of the invention are (1'R,2'R,4'R)-4-bromo-5'-methyl-2'-(prop-1-en-2-yl)-1',2',3',4'-tetrahydro-[1,1'-biphenyl]-2,4',6-triol, which will be referred to as Isomer 1 of Compound I, and (1'R,2'R,4'S)-4-bromo-5'-methyl-2'-(prop-1-en-2-yl)-1',2',3',4'-tetrahydro-[1,1'-biphenyl]-2,4',6-triol, which will be referred to as Isomer 2 of Compound I, and Isomers 1 and 2 will be referred to collectively as the compound of Formula I or Compound I throughout.

Analytical data of Isomer 1 of Compound I is as follows: ¹H NMR (400 MHz, DMSO) δ 9.37 (s, 2H), 6.39 (s, 2H), 5.17 (s, 1H), 4.63 - 4.58 (m, 1H), 4.51 (s, 1H), 4.47 - 4.46 (m, 1H), 4.08 - 4.01 (m, 1H), 3.87 (s, 1H), 3.74 - 3.72 (m, 1H), 2.01 (d, J=5.0 Hz, 1H), 1.70 - 1.53 (m, 7H).

Analytical data of Isomer 2 of Compound I is as follows: ¹H NMR (400 MHz, DMSO) δ 9.53 (s, 1H), 9.32 (s, 1H), 6.43 - 6.36 (m, 2H), 5.07 - 5.05 (m, 1H), 4.62 (d, J=6.7 Hz, 1H), 4.49 - 4.44 (m, 2H), 4.12 - 4.06 (m, 1H), 3.88 - 3.84 (m, 1H), 3.20 - 3.12 (m, 1H), 1.90 (ddd, J=2.4, 5.7, 12.0 Hz, 1H), 1.66 - 1.63 (m, 3H), 1.63 - 1.55 (m, 4H).

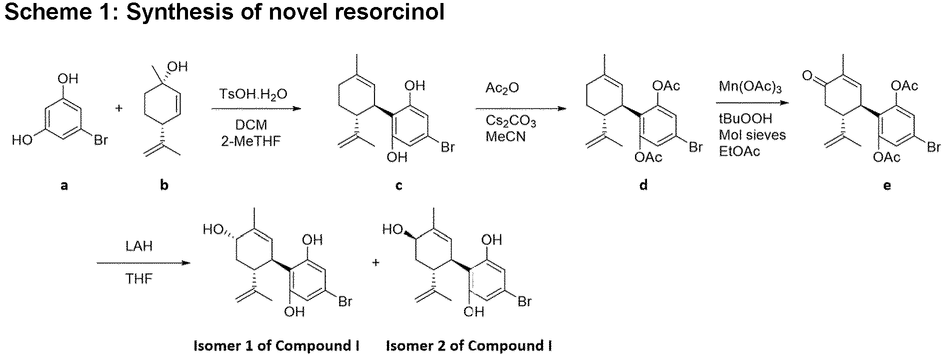

| **Compound** | **Name** |
|---|---|
| **a** | 5-bromobenzene-1,3-diol |
| **b** | 4-isopropenyl-1-methyl-cyclohex-2-en-1-ol |
| **c** | 5-Bromo-2-[6-isopropenyl-3-methyl-cyclohex-2-en-1-yl]benzene-1,3-diol |
| **d** | [3-Acetoxy-5-bromo-2-[6-isopropenyl-3-methyl-cyclohex-2-en-1-yl]phenyl] acetate |
| **e** | [3-Acetoxy-5-bromo-2-[6-isopropenyl-3-methyl-4-oxo-cyclohex-2-en-1-yl]phenyl] acetate |

### Step 1: Formation of Compound c

A solution of 5-bromobenzene-1,3-diol (20.88 g, 0.110 mol, 1.00 eq) and p-toluenesulfonic acid monohydrate (10.51 g, 55.2 mmol, 0.500 eq) in a mixture of 2-methyltetrahydrofuran (132 mL) and dichloromethane (465 mL) was cooled to 0°C with an ice/brine bath under nitrogen. (4R)-4-isopropenyl-1-methyl-cyclohex-2-en-1-ol (13 mL, 77.5 mmol, 0.701 eq) was added and the resulting solution stirred for 5 minutes. The cooling bath was removed and the colourless solution stirred for 2 hours, warming to 20°C. The mixture was diluted with dichloromethane (200 mL) and basified to pH 8 by careful addition of 300 mL saturated aqueous NaHCO₃. The organic layer was separated and washed with water (50 mL), saturated brine (50 mL) then dried (MgSO₄), filtered and concentrated *in vacuo* to give a colourless gum. This was purified by column chromatography on silica gel (800 g, Interchim cartridge), eluting with 0-50% diethyl ether in cyclohexane to give 5-bromo-2-[(1*R*,6*R*)-6-isopropenyl-3-methyl-cyclohex-2-en-1-yl]benzene-1,3-diol (2.53 g, ca 6% yield) as a colourless gum/glass. This was repurified by column chromatography on silica gel (40 g, 15 micron Interchim column), eluting with 5-20% diethyl ether in cyclohexane vacuo to give 5-bromo-2-[(1*R*,6*R*)-6-isopropenyl-3-methyl-cyclohex-2-en-1-yl]benzene-1,3-diol (0.92 g) as well as some impure material.

The initial column also yielded recovered 5-bromobenzene-1,3-diol (8.17 g, ca 39% recovery) as a colourless gum that solidified on standing. This was dissolved in a mixture of 2-methyltetrahydrofuran (55 mL) and dichloromethane (185 mL), treated with (4*R*)-4-isopropenyl-1-methyl-cyclohex-2-en-1-ol (4.9 mL, 30.3 mmol, 0.701 eq), cooled to 0°C with an ice/brine bath under nitrogen. *p*-Toluenesulfonic acid monohydrate (4.11 g, 21.6 mmol, 0.500 eq) was added and the resulting solution was stirred for 5 minutes. The cooling bath was removed and the colourless solution was stirred for 2 hours, warming to 20°C. The mixture was diluted with dichloromethane (100 mL) and basified to pH 8 by careful addition of 300 mL saturated aqueous sodium hydrogen carbonate. The organic layer was separated and washed with water (50 mL), saturated brine (50 mL) then dried (MgSO₄), filtered and concentrated *in vacuo* to give a colourless gum. The residue was purified by column chromatography on silica gel (40 g Interchim cartridge), eluting with 0-50% diethyl ether in cyclohexane to give the title compound as a colourless gum (1.33 g, 7% yield, 81% LCMS purity). This was combined with impure material from the first reaction and purified by column chromatography on silica gel (40 g Interchim cartridge), eluting with 5-20% diethyl ether in cyclohexane to give 5-bromo-2-[(1*R*,6*R*)-6-isopropenyl-3-methyl-cyclohex-2-en-1-yl]benzene-1,3-diol as a colourless gum (2.10 g, 91% LCMS purity).

The total yield of 5-bromo-2-[(1R,6R)-6-isopropenyl-3-methyl-cyclohex-2-en-1-yl]benzene-1,3-diol obtained was 3.02 g (9.34 mmol, 8.5%).

Compound c analytical data: ¹H NMR (400 MHz, DMSO) δ 9.37 (s, 2H), 6.38 (s, 2H), 5.08 (s, 1H), 4.49 (d, J=2.8 Hz, 1H), 4.44 (dd, J=1.6, 2.8 Hz, 1H), 3.86 - 3.83 (m, 1H), 3.06 - 2.98 (m, 1H), 2.12 - 2.07 (m, 1H), 1.96 - 1.92 (m, 1H), 1.63 - 1.59 (m, 8H).

### Step 2: Formation of Compound d

5-Bromo-2-[(1R,6R)-6-isopropenyl-3-methyl-cyclohex-2-en-1-yl]benzene-1,3-diol (1305 mg, 4.04 mmol, 1.00 eq) in acetonitrile (30 mL) was treated with cesium carbonate (3289 mg, 10.1 mmol, 2.50 eq) followed by acetic anhydride (0.95 mL, 10.1 mmol, 2.50 eq). The reaction mixture was stirred for 1 hour then partitioned between ethyl acetate (100 mL) and water (100 mL). The layers were separated, the aqueous layer was extracted with ethyl acetate (100 mL) and the combined organic phases were filtered through hydrophobic filter paper and concentrated *in vacuo* to give the title compound as an orange gum (1.60 g, 93.4%). This was used without further purification in the next reaction.

Compound **d** analytical data:¹H NMR (400 MHz, CDCl3) δ , 7.07 (s, 2H), 5.15 (s, 1H), 4.56 (s, 1H), 4.44 **-** 4.44 (m, 1H), 3.55 - 3.50 (m, 1H), 2.67 - 2.60 (m, 1H), 2.24 - 2.16 (m, 7H), 2.07 - 2.00 (m, 1H), 1.84 - 1.64 (m, 5H), 1.58 - 1.57 (m, 3H).

### Step 3: Formation of Compound e

[3-Acetoxy-5-bromo-2-[(1*R*,6*R*)-6-isopropenyl-3-methyl-cyclohex-2-en-1-yl]phenyl] acetate (1560 mg, 3.83 mmol, 1.00 eq) and molecular sieves (3Å, 5000mg) in ethyl acetate (31.92 mL) were treated with manganese(III) acetate dihydrate (103 mg, 0.383 mmol, 0.100 eq) followed by 5.5 M tert-butyl hydroperoxide solution in nonane (3.5 mL, 19.2 mmol, 5.00 eq) The reaction mixture was stirred at 20°C overnight then diluted with ethyl acetate (50 mL) and filtered through a pad of celite. The filtrate was washed with saturated aqueous sodium thiosulfate (30 mL) and water (30 mL), separated, dried (MgSO₄), filtered and concentrated *in vacuo.* The residue was purified by column chromatography on silica gel, eluting with 0-100% diethyl ether in cyclohexane to give the title compound as an off-white solid (561 mg, 34.8%).

Compound **d** analytical data: ¹H NMR (400 MHz, DMSO) δ 7.39 (s, 2H), 6.38 (t, J=1.8 Hz, 1H), 4.56 (s, 1H), 4.50 (s, 1H), 4.04 (td, J=2.3, 10.6 Hz, 1H), 3.20 - 3.11 (m, 1H), 2.85 - 2.76 (m, 1H), 2.35 - 2.19 (m, 7H), 1.71 (dd, J=1.5, 2.5 Hz, 3H), 1.62 (s, 3H).

### Step 4: Formation of Isomer 1 of Compound I and Isomer 2 of Compound I

To a solution of [3-acetoxy-5-bromo-2-[(1*R*,6*R*)-6-isopropenyl-3-methyl-4-oxo-cyclohex-2-en-1-yl]phenyl] acetate (1.10 g, 2.61 mmol, 1.00 eq) in methyl alcohol (25.0 mL) at 0 °C was added sodium borohydride (0.22 g, 5.74 mmol, 2.20 eq) in four equal portions over a period of 30 minutes and the mixture was allowed to warm to room temperature overnight. The reaction mixture was concentrated *in vacuo* and the residue partitioned between ethyl acetate (75 mL) and water (75 mL). The layers were separated and the organic phase was washed with brine (50 mL), dried (MgSO₄) and concentrated *in vacuo.* The residue was purified by column chromatography on silica gel eluting with 0-80% ethyl acetate in cyclohexane to give (1'*R*,2'*R*,4'*R*)-4-bromo-5'-methyl-2'-(prop-1-en-2-yl)-1',2',3',4'-tetrahydro-[1,1'-biphenyl]-2,4',6-triol (200 mg, 22.5%) as a white solid and (1'*R*,2'*R*,4'*S*)-4-bromo-5'-methyl-2'-(prop-1-en-2-yl)-1',2',3',4'-tetrahydro-[1,1'-biphenyl]-2,4',6-triol (508 mg, 57.4%) as a white solid.

### Example 2: Evaluation of Novel Resorcinol for Anticonvulsant Activity using the Maximal Electroshock Seizure Threshold (MEST) Test in the Mouse Using Minimal Sample Sizes (mini MEST)

The efficacy of the novel resorcinol according to the compound of Formula I was tested in a novel mouse model of generalised seizure, the mini-MEST (maximal electroshock seizure threshold) test, which uses lower n numbers than typically used.

The maximal electroshock seizure threshold (MEST) test is widely utilized preclinically to evaluate pro- or anti-convulsant properties of test compounds (Loscher et al., 1991).

In the MEST test the ability of a drug to alter the seizure threshold current required to induce hind limb tonic extensor convulsions is measured according to an "up and down" method of shock titration (Kimball et al., 1957). An increase in seizure threshold is indicative of anti-convulsant effect. Antiepileptic drugs including the sodium channel blockers (e.g. lamotrigine) with clinically proven efficacy against generalised tonic-clonic seizures all exhibit anti-convulsant properties in this test in the mouse.

Conversely, a reduction in seizure threshold is indicative of a pro-convulsant effect as observed with known convulsant agents such as picrotoxin.

The ability of a test compound to alter the stimulus intensity, expressed as current (mA), required to induce the presence of tonic hind limb extensor convulsions, is assessed in the MEST. The outcome of the presence (+) or absence (0) of tonic hind limb extensor convulsions observed from a current to produce tonic hind limb extension in 50% of animals in the treatment group (CC₅₀) determines the seizure threshold for the treatment group and the effects were then compared to the CC₅₀ of the vehicle control group.

### Methods

### Study Details:

Naïve mice were acclimatised to the procedure room in their home cages for up to 7 days, with food and water available ad libitum.

All animals were weighed at the beginning of the study and randomly assigned to treatment groups based on a mean distribution of body weight across groups. All animals were dosed at 10 mL/kg via intraperitoneal (i.p) injection, with either vehicle, test compound at 50 mg/kg, or diazepam at 2.5 mg/kg.

Animals were individually assessed for the production of a tonic hind limb extensor convulsion at 30 min post-dose for vehicle, test compound and diazepam, from a single electroshock.

The first animal within a treatment group was given a shock at the expected or estimated CC₅₀ current. For subsequent animals, the current was lowered or raised depending on the convulsions outcome from the preceding animal in log scale intervals.

Data generated from each treatment group were used to calculate the CC₅₀ ± SEM values for the treatment group.

### Test Compounds:

Vehicle: (5% ethanol, 10% solutol in 85% Saline) was prepared as follows: 1 mL of ethanol, 2 mL of solutol were warmed to 60°C, in 17 mL of saline (1:2:17).

Positive control: diazepam was used at 2.5mg/kg.

The test compound, described herein as Compound I, is as shown as Formula I. Test compound was administered at 50mg/kg (i.p.) in a 1:2:17 ethanol:solutol:0.9% saline formulation.

### Sample Collection:

Each animal was humanely killed immediately after production of a convulsion by destruction of the brain from striking the cranium, followed by the confirmation of permanent cessation of the circulation from decapitation under The Humane Killing of Animals under Schedule 1 to the Animals (Scientific Procedures) Act 1986. Terminal blood and brain collection were performed following decapitation.

Blood was collected in Lithium-heparin tubes and centrifuged at 4°C for 10 minutes at 1500 x g. The resulting plasma was removed (>100 µL) and split into 2 aliquots of 0.5 mL Eppendorf tubes containing 10 µL of ascorbic acid (100 mg/mL) for stabilisation. Brains were removed, washed in saline and halved. Each half was placed into separate 2 mL screw cap cryovials, weighed and frozen on cardice.

### Statistical analysis:

The data for each treatment group were recorded as the number of +'s and 0's at each current level employed and this information is then used to calculate the CC₅₀ value (current required for 50% of the animals to show seizure behaviour) ± standard error.

Test compound effects were also calculated as percentage change in CC₅₀ from the vehicle control group.

Significant difference between drug-treated animals and controls were assessed according to Litchfield and Wilcoxon (1949).

### Results

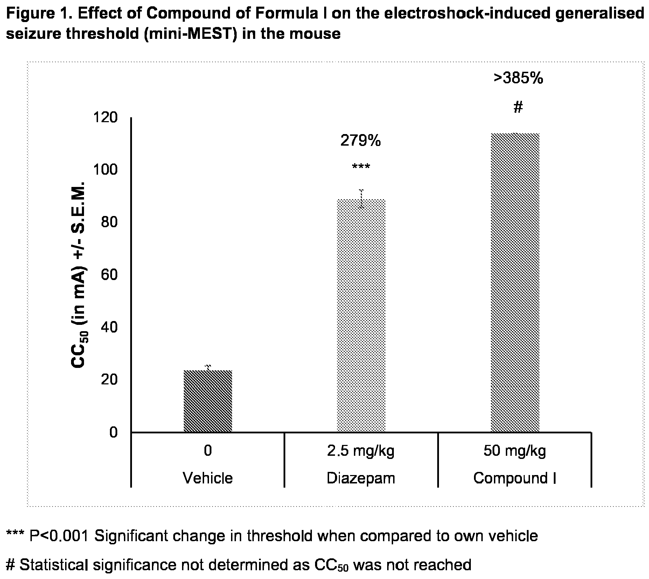

Figure 1 and Table 1 describe the data produced in this experiment.

In the vehicle group, the CC₅₀ value was calculated to be 23.5 mA.

In the diazepam (2.5 mg/kg) treated group, administered i.p. 30 minutes before the test, the CC₅₀ value was 89.0 mA. This result was statistically significant (p<0.001) compared to vehicle control.

The test compound, administered i.p. 30 minutes before the test, produced a clear increase in seizure threshold as compared to vehicle, with CC₅₀ > 114 mA for 50mg/kg; an exact value was not calculated as a "+" tonic hindlimb convulsion was not seen within the 6 animals tested. Although CC₅₀ was not determined, Compound I showed a clear increase in seizure threshold in the mini-MEST. Clear activity of Compound I was demonstrated as animals in this treatment group did not have any convulsions.

Such data are indicative that this compound will be of therapeutic benefit.

**Table 1: Evaluation of effect of Compound I in the mini-MEST test**

| **Treatment** | **Dose (mg/kg)** | **Test time post dose (min)** | **N** | **CC₅₀ +/- SEM** | **Significance** | **% change from vehicle** |
|---|---|---|---|---|---|---|
| **Vehicle** | - | 30 | 6 | 23.5+/-1.9 | - | - |
| **Diazepam** | 2.5 | 30 | 6 | 89.0+/-3.4 | P<0.001 | 279% |
| **Compound I** | 50 | 30 | 6 | >114 | # | >385 % |

| | | | | | | |
|---|---|---|---|---|---|---|
| # Statistical significance not determined as CC50 was not reached | | | | | | |

### Conclusions

This data produced using the mini-MEST model demonstrates a strong therapeutic effect for Compound I, with CC₅₀ increasing by more than 385% compared to vehicle control, a percentage change that is even higher than one produced by the positive control.

This data is significant as it provides heretofore unknown evidence that this novel resorcinol may be of therapeutic value.

### Example 3: Evaluation of Novel Resorcinol for Anticonvulsant Activity using the Maximal Electroshock Seizure Threshold (MEST) Test in the Mouse

The efficacy of the novel resorcinol according to the compound of Formula I was tested in a mouse model of generalised seizure, the maximal electroshock seizure threshold (MEST) test.

The maximal electroshock seizure threshold (MEST) test is widely utilized preclinically to evaluate pro- or anti-convulsant properties of test compounds (Loscher et al., 1991).

In the MEST test the ability of a drug to alter the seizure threshold current required to induce hind limb tonic extensor convulsions is measured according to an "up and down" method of shock titration (Kimball et al., 1957). An increase in seizure threshold is indicative of anti-convulsant effect. Antiepileptic drugs including the sodium channel blockers (e.g. lamotrigine) with clinically proven efficacy against generalised tonic-clonic seizures all exhibit anti-convulsant properties in this test in the mouse.

Conversely, a reduction in seizure threshold is indicative of a pro-convulsant effect as observed with known convulsant agents such as picrotoxin.

The ability of a test compound to alter the stimulus intensity, expressed as current (mA), required to induce the presence of tonic hind limb extensor convulsions, is assessed in the MEST. The outcome of the presence (+) or absence (0) of tonic hind limb extensor convulsions observed from a current to produce tonic hind limb extension in 50% of animals in the treatment group (CC₅₀) determines the seizure threshold for the treatment group and the effects were then compared to the CC₅₀ of the vehicle control group.

### Methods

### Study Details:

Naïve mice were acclimatised to the procedure room in their home cages for up to 7 days, with food and water available ad libitum.

All animals were weighed at the beginning of the study and randomly assigned to treatment groups based on a mean distribution of body weight across groups. All animals were dosed at 10 mL/kg via intraperitoneal (i.p) injection, with either vehicle, test compound at 1, 5, 20, 50, 100 and 200 mg/kg or diazepam at 2.5 mg/kg.

Animals were individually assessed for the production of a tonic hind limb extensor convulsion at 60 min post-dose for vehicle, 15-60 min post-dose for test compound and 30 min post-dose for diazepam, from a single electroshock.

The first animal within a treatment group was given a shock at the expected or estimated CC₅₀ current. For subsequent animals, the current was lowered or raised depending on the convulsions outcome from the preceding animal, in intervals of 5 mA.

Data generated from each treatment group were used to calculate the CC₅₀ ± SEM values for the treatment group.

### Test Compounds:

Vehicle: (5% ethanol, 5% Kolliphor^{®} EL, 90% Saline) was prepared as follows: 2 mL of ethanol, 2 mL of Kolliphor^{®} EL were warmed to 60°C, in 36 mL of saline (1:1:18).

Positive control: diazepam was used at 2.5mg/kg.

The test compound, described herein as Compound I, is as shown as Formula I. Test compound was administered at 1, 5, 20, 50, 100 and 200 mg/kg (i.p.) in a 1:2:17 ethanol:solutol:0.9% saline formulation.

### Sample Collection:

Each animal was humanely killed immediately after production of a convulsion by destruction of the brain from striking the cranium, followed by the confirmation of permanent cessation of the circulation from decapitation under The Humane Killing of Animals under Schedule 1 to the Animals (Scientific Procedures) Act 1986. Terminal blood and brain collection were performed following decapitation.

Blood was collected in Lithium-heparin tubes and centrifuged at 4°C for 10 minutes at 1500 x g. The resulting plasma was removed (>100 µL) and split into 2 aliquots of 0.5 mL Eppendorf tubes containing 100 µL of ascorbic acid (100 mg/mL) for stabilisation. Brains were removed, washed in saline and halved. Each half was placed into separate 2 mL screw cap cryovials, weighed and frozen on cardice.

### Statistical analysis

The data for each treatment group were recorded as the number of +'s and 0's at each current level employed and this information is then used to calculate the CC₅₀ value (current required for 50% of the animals to show seizure behaviour) ± standard error.

Test compound effects were also calculated as percentage change in CC₅₀ from the vehicle control group.

Significant difference between drug-treated animals and controls were assessed according to Litchfield and Wilcoxon (1949).

### Results

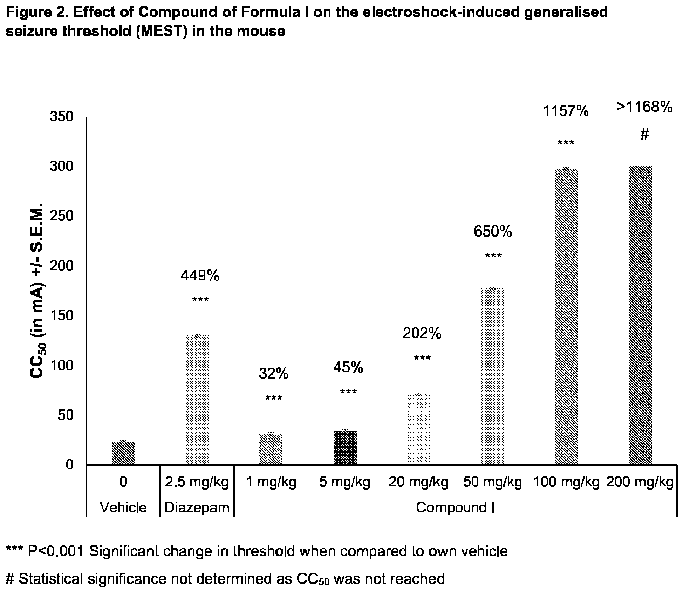

Figure 2 and Table 2 describe the data produced in this experiment.

In the vehicle group, the CC₅₀ value was calculated to be 23.7mA.

In the diazepam (2.5 mg/kg) treated group, administered i.p. 30 minutes before the test, the CC₅₀ value was 130.0mA. This result was statistically significant (p<0.001) compared to the vehicle control.

The test compound, administered i.p. 15-60 minutes before the test, produced a statistically significant CC₅₀ value compared to vehicle at 1, 5, 20, 50 and 100 mg/kg, which suggests this compound exhibits anticonvulsive properties.

At 200mg/kg, Compound I produced a clear increase in seizure threshold as compared to vehicle, with CC₅₀ > 300 mA; an exact value was not calculated as a "+" tonic hindlimb convulsion was not seen within the 12 animals tested. Although CC₅₀ was not determined, 200 mg/kg showed a clear increase in seizure threshold in the MEST.

Such data are indicative that this compound will be of therapeutic benefit.

**Table 2: Evaluation of effect of Compound I in the MEST test**

| **Treatment** | **Dose (mg/kg)** | **Test time post dose (min)** | **N** | **CC₅₀ +/- SEM** | **Significance** | **% change from vehicle** |
|---|---|---|---|---|---|---|
| **Vehicle** | - | 60 | 12 | 23.7 ± 0.4 | - | - |
| **Diazepam** | 2.5 | 30 | 12 | 130.0 ± 1.1 | P<0.001 | 449% |
| **Compound I** | 1 | 15 | 12 | 31.3 ± 1.5 | P<0.001 | 32% |
| **Compound I** | 5 | 15 | 12 | 34.2 ± 1.5 | P<0.001 | 45% |
| **Compound I** | 20 | 30 | 12 | 71.5 ± 1.0 | P<0.001 | 202% |
| **Compound I** | 50 | 30 | 12 | 177.5 ± 0.7 | P<0.001 | 650% |
| **Compound I** | 100 | 60 | 12 | 297.5 ± 0.7 | P<0.001 | 1157% |
| **Compound I** | 200 | 60 | 12 | >300.0 | # | >1168% |

| | | | | | | |
|---|---|---|---|---|---|---|
| # Significance not determined as CC₅₀ not reached | | | | | | |

### Conclusions

These data produced using the MEST model demonstrate a therapeutic effect for the compound of Formula I, with CC₅₀ increasing at all doses compared to vehicle control.

These data are significant as they provide heretofore unknown evidence that this novel resorcinol may be of therapeutic value.

Clearly the compound produced a dose-related increase in MEST, suggesting that this compound exhibits anticonvulsive properties. Significant effects were observed when compared to vehicle.

### Example 4: Evaluation of Novel Resorcinol for Anticonvulsant Activity using the DBA/2 mouse Audiogenic Seizure Test

The DBA/2 audiogenic seizure test, which detects anticonvulsant activity, follows that described by Dürmüller et al. (NeuroReport, vol. 4, no. 6, pages 683-686, 1993).

### Methods

### Study Details:

60 male DBA/2 mice (3 - 4 weeks old) were included in the experiment (weight range 8 - 11 g at the beginning of the experiment). Animals were acclimatized to the test facility for 1 day after delivery and randomly housed in groups of 5 in macrolon cages on wood litter with free access to food and water.

The animal house was maintained under artificial lighting (12 hours) between 7:00 and 19:00 in a controlled ambient temperature of 22 ± 2°C, and relative humidity between 30-70%.

Mice were individually transferred (at 3 - 5 minutes intervals) from the preparation room into an adjacent experimental room and body temperature was measured using a rectal thermometer. Immediately after, they were placed in a Plexiglas jar (Diameter = 40 cm; Height = 35 cm) mounted with an electric bell. Upon activating the bell, the occurrences and latencies to wild running fits, clonic and tonic seizures were measured. Deaths were also recorded. The bell was activated until a tonic seizure occured or for a maximum of 60 seconds.

The experiment included 6 groups (10 mice were studied per group). Experimenters were blinded to the treatment groups.

The test substance was evaluated at 4 doses, administered i.p. (10 mL/kg) 30 or 60 minutes before the test, and compared with a vehicle control group. Doses and pretreatment times were based on previous pharmacokinetic data.

**Table 3: Treatment schedule**

| **Group** | **Number of animals** | **Treatment** | **Pretreatment time** | **Dose-level (mg/kg)** | **Concentration (mg/mL)** | **Administration Volume (mL/kg)** |
|---|---|---|---|---|---|---|
| **1** | 10 | Valproate | 30 min | 180 | 18 | 10 |
| **2** | 10 | Vehicle | 30 min | 0 | 0 | 10 |
| **3** | 10 | Compound I | 30 min | 20 | 2 | 10 |
| **4** | 10 | Compound I | 30 min | 50 | 5 | 10 |
| **5** | 10 | Compound I | 60 min | 100 | 10 | 10 |
| **6** | 10 | Compound I | 60 min | 200 | 20 | 10 |

### Test Compounds:

Test compound: Compound I dissolved in 5% ethanol, 10% Kolliphor HS15, 85% physiological saline.

Vehicle control: 5% ethanol, 10% Kolliphor HS15, 85% physiological saline.

Reference: Valproate (180 mg/kg), dissolved in 5% ethanol, 10% Kolliphor HS15, 85% physiological saline.

### Statistical analysis

Data was tested for normality using d'Agostino-Pearson and found to not follow a normal distribution. Statistical outliers were not identified or removed because no behavioural outliers were identified.

Quantitative data (latencies) with the test substance were analyzed by comparing treated groups with vehicle control using Kruskal-Wallis test followed by Dunn's multiple comparisons test. Quantitative data with the reference substance were analyzed using Mann-Whitney U test.

Quantal data (frequencies) were analyzed by comparing treated groups with vehicle control using Fisher's Exact Probability tests.

### Results

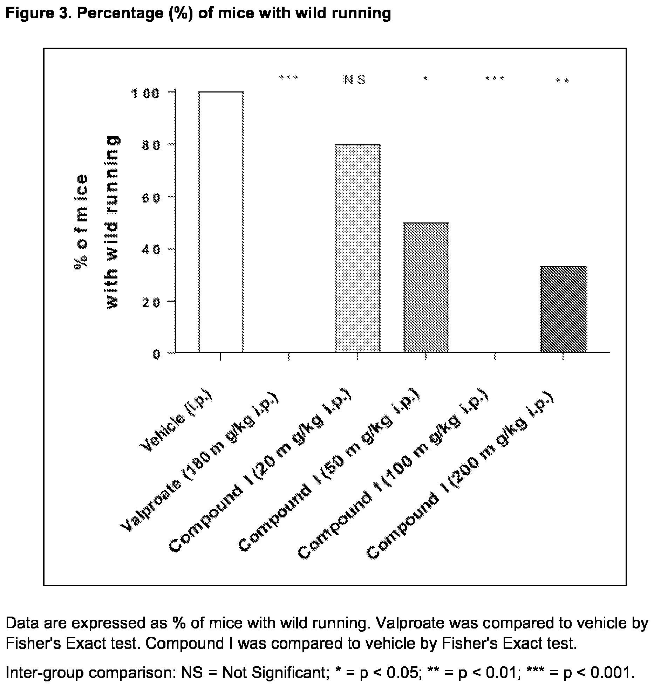

Figures 3-8 and Tables 4-7 describe the data produced in this experiment.

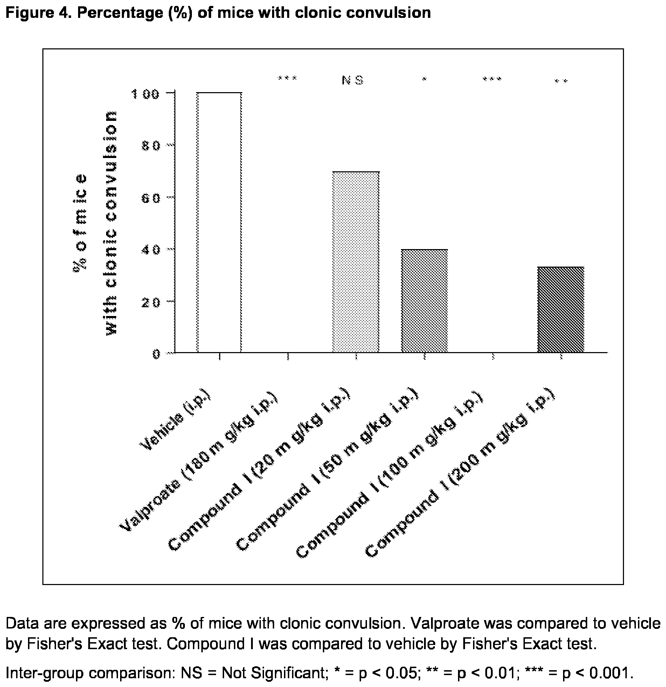

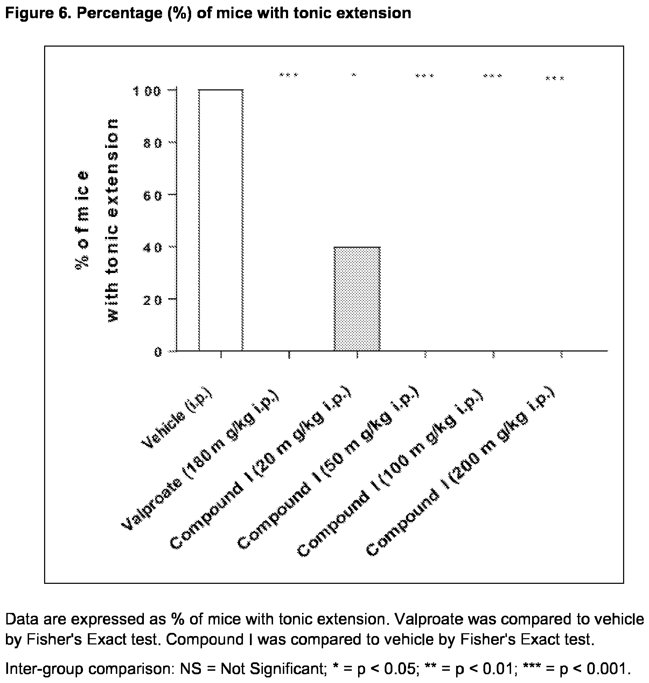

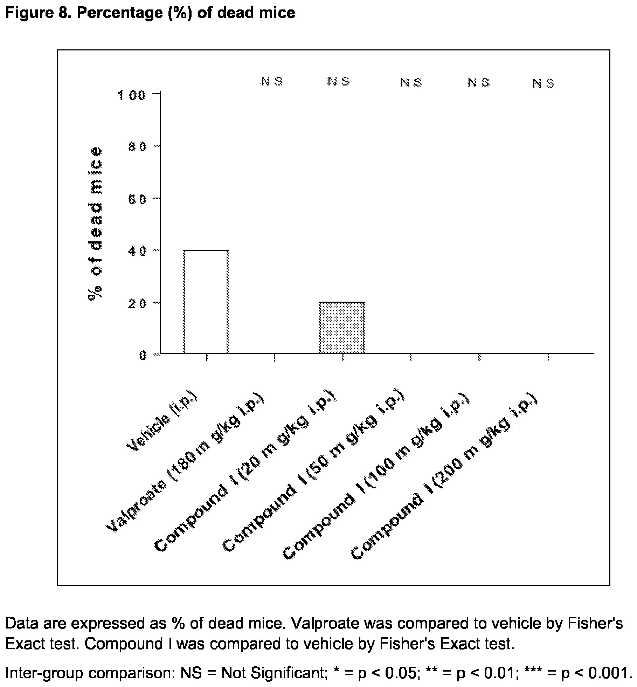

As for the negative control of the experiment, in vehicle controls administered i.p. 30 minutes before the test, all mice displayed wild running (see Figure 3, furthest left column at 100%), clonic (Figure 4 shows 100%) and tonic convulsions (Figure 6). 4 out of the 10 mice tested died (Figure 8). Median latency to wild running was 3.3 s (IQR 2.7-4.0) (Table 4), median latency to clonic convulsion was 6.6 s (IQR 6.1-7.7) (Table 5), and median latency to tonic convulsion was 10.7 s (IQR 9.6-12.1) (Table 6).

As for the positive control of the experiment, valproate (180 mg/kg), administered i.p. 30 minutes before the test, suppressed wild running (see Figure 3, second left column at 0%), clonic convulsions (Figure 4 shows 0%) and tonic convulsions (Figure 6), as compared with vehicle controls (-100%, p<0.001) for each parameter. Valproate significantly increased the corresponding latencies (+1718%, +809%, and +461%, respectively, p<0.001 for each parameter) (Tables 4-6), as compared with vehicle controls. No deaths were observed in animals dosed with valproate (Figure 8 and Table 9). No adverse signs were noted in animals dosed with valproate.

Four mice in each of the groups dosed Compound I at 100 and 200 mg/kg had a rectal temperature below 35.0 °C. These mice were removed from all analyses. Tremor was noted for 5 mice treated at 100 mg/kg and 3 mice treated at 200 mg/kg.

Compound I at 50, 100 and 200 mg/kg significantly decreased the number of mice showing wild running (-50%, p<0.05; -100%, p<0.001 and -80%, p<0.01, respectively), see Figure 3.

There was a statistically significant main effect of Compound I dose on latency to wild running as compared to vehicle (p < 0.001, see Table 4). Dunn's multiple comparisons test showed that Compound I at 50, 100 and 200 mg/kg significantly increased latency to wild running as compared to vehicle (mean rank -19.10, p < 0.01; - 26.55, p < 0.001 and -23.22, p < 0.001, respectively).

Compound I at 50, 100 and 200 mg/kg, significantly decreased the number of mice showing clonic convulsions (-60%, p<0.05; -100%, p<0.001 and -80%, p<0.01, respectively), see Figure 4.

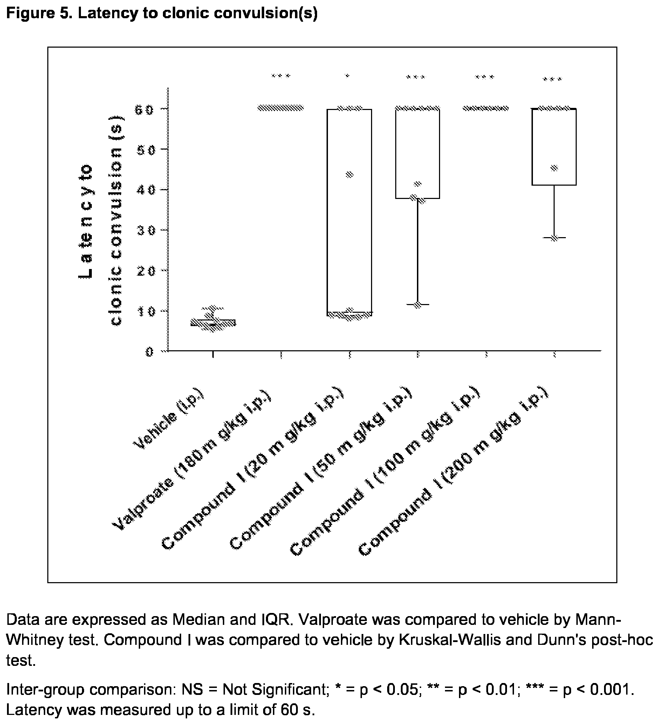

There was a statistically significant main effect of Compound I dose on latency to clonic convulsions, as compared to vehicle (p < 0.001, Figure 5). Dunn's multiple comparisons test showed that Compound I at 20, 50, 100 and 200 mg/kg significantly increased latency to clonic convulsion as compared to vehicle (mean rank -13.10, p < 0.05; -21.20, p < 0.001 ; -26.70, p < 0.001 and -22.53, p < 0.001, respectively).

As shown by Figure 6, at all doses, Compound I, significantly decreased the number of mice showing tonic convulsions (-60%, p<0.05 at 20 mg/kg; -100%, p<0.001 at other doses).

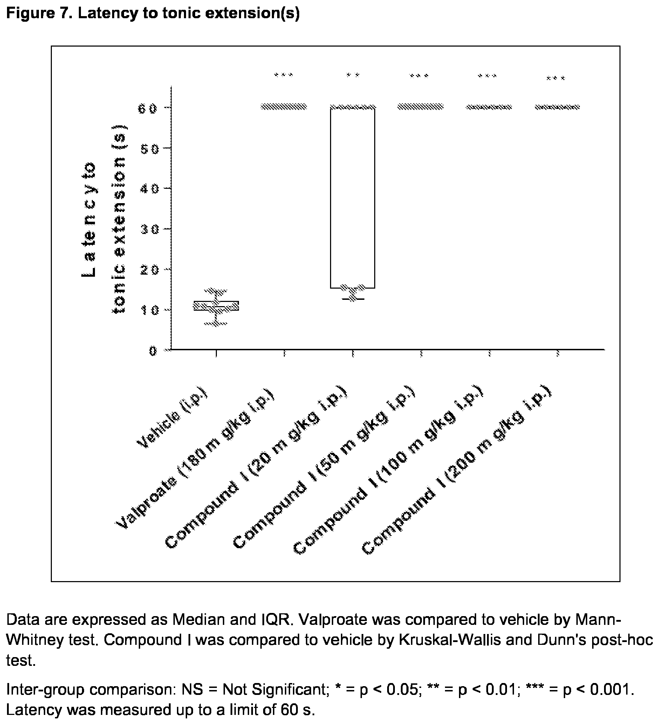

There was a statistically significant main effect of Compound I dose on latency to tonic convulsions (Figure 7), as compared to vehicle (p < 0.001). Dunn's multiple comparisons test showed that Compound I at 20, 50, 100 and 200 mg/kg significantly increased latency to tonic convulsion as compared to vehicle (mean rank -16.20, p < 0.01 at 20 mg/kg; -22.80, p < 0.001 for the last three doses).

As evidenced by Figure 8 and Table 7, Compound I did not affect the number of deaths at any doses.

**Table 4: Latency to wild running**

| **Treatment** | **Wild running** | | | | | | | | | |
|---|---|---|---|---|---|---|---|---|---|---|
| | **Presence** | | | | **Latency (s)** | | | | | |
| | **Total** | **%** | **Compared with Vehicle (i.p.)** | | **Median** | **IQR** | | **Compared with Vehicle (i.p.)** | | |
| | | | | | | **Q1** | **Q3** | | | |
| | | | **(1)** | **%** | | | | **(2)** | **(3)** | **%** |
| **Vehicle (i.p.)** | 10/10 | 100 | | | 3.3 | 2.7 | 4.0 | | | |
| **Valproate (180 mg/kg i.p.)** | 0/10 | 0 | *** | -100% | 60.0 | 60.0 | 60.0 | *** | | +1718% |
| **Compound I (20 mg/kg i.p.)** | 8/10 | 80 | NS | -20% | 5.1 | 3.7 | 44.0 | | NS | +55% |
| **Compound I (50 mg/kg i.p.)** | 5/10 | 50 | * | -50% | 33.8 | 5.6 | 60.0 | *** | ** | +924% |
| **Compound I (100 mg/kg i.p.)** | 0/6 | 0 | *** | -100% | 60.0 | 60.0 | 60.0 | | *** | +1718% |
| **Compound I (200 mg/kg i.p.)** | 2/6 | 33 | ** | -80% | 60.0 | 35.0 | 60.0 | | *** | +1718% |

| | | | | | | | | | | |
|---|---|---|---|---|---|---|---|---|---|---|
| NS = Not Significant; * = p < 0.05; ** = p < 0.01; *** = p < 0.001. | | | | | | | | | | |

**Table 5: Latency to clonic convulsion**

| **Treatment** | **Clonic convulsion** | | | | | | | | | |
|---|---|---|---|---|---|---|---|---|---|---|
| | **Presence** | | | | **Latency (s)** | | | | | |
| | **Total** | **%** | **Compared with Vehicle (i.p.)** | | **Median** | **IQR** | | **Compared with Vehicle (i.p.)** | | |
| | | | | | | **Q1** | **Q3** | | | |
| | | | **(1)** | **%** | | | | **(2)** | **(3)** | **%** |
| **Vehicle (i.p.)** | 10/10 | 100 | | | 6.6 | 6.1 | 7.7 | | | |
| **Valproate (180 mg/kg i.p.)** | 0/10 | 0 | *** | -100% | 60.0 | 60.0 | 60.0 | *** | | +809% |
| **Compound** I **(20 mg/kg i.p.)** | 7/10 | 70 | NS | -30% | 9.5 | 8.7 | 60.0 | *** | * | +44% |
| **Compound I (50 mg/kg i.p.)** | 4/10 | 40 | * | -60% | 60.0 | 37.7 | 60.0 | | *** | +809% |
| **Compound I (100 mg/kg i.p.)** | 0/6 | 0 | *** | -100% | 60.0 | 60.0 | 60.0 | | *** | +809% |
| **Compound I (200 mg/kg i.p.)** | 2/6 | 33 | ** | -80% | 60.0 | 41.0 | 60.0 | | *** | +809% |

| | | | | | | | | | | |
|---|---|---|---|---|---|---|---|---|---|---|
| NS = Not Significant; * = p < 0.05; ** = p < 0.01; *** = p < 0.001. | | | | | | | | | | |

**Table 6: Latency to tonic extension**

| **Treatment** | **Tonic extension** | | | | | | | | | |
|---|---|---|---|---|---|---|---|---|---|---|
| | **Presence** | | | | | | | | | |
| | **Total** | **%** | **Compared with** | | **Median** | **IQR** | | **Compared with** | | |
| | | | **Vehicle (i.p.)** | | | **Q1** | **Q3** | **Vehicle (i.p.)** | | |
| | | | **(1)** | **%** | | | | **(2)** | **(3)** | **%** |
| **Vehicle (i.p.)** | 10/10 | 100 | | | 10.7 | 9.6 | 12.1 | | | |
| **Valproate (180 mg/kg i.p.)** | 0/10 | 0 | *** | -100% | 60.0 | 60.0 | 60.0 | *** | | +461% |
| **Compound I (20 mg/kg i.p.)** | 4/10 | 40 | * | -60% | 60.0 | 15.2 | 60.0 | *** | ** | +461% |
| **Compound I (50 mg/kg i.p.)** | 0/10 | 0 | *** | -100% | 60.0 | 60.0 | 60.0 | | *** | +461% |
| **Compound I (100 mg/kg i.p.)** | 0/6 | 0 | *** | -100% | 60.0 | 60.0 | 60.0 | | *** | +461% |
| **Compound I (200 mg/kg i.p.)** | 0/6 | 0 | *** | -100% | 60.0 | 60.0 | 60.0 | | *** | +461% |

| | | | | | | | | | | |
|---|---|---|---|---|---|---|---|---|---|---|
| NS = Not Significant; * = p < 0.05; ** = p < 0.01; *** = p < 0.001. | | | | | | | | | | |

**Table 7: Percentage (%) of death**

| **Treatment** | Death | | | |
|---|---|---|---|---|
| | **Presence** | | | |
| | **Total** | **%** | **Compared with Vehicle (i.p.)** | |
| | | | **(1)** | **%** |
| **Vehicle (i.p.)** | 4/10 | 40 | | |
| **Valproate (180 mg/kg i.p.)** | 0/10 | 0 | NS | -100% |
| **Compound I (20 mg/kg i.p.)** | 2/10 | 20 | NS | -50% |
| **Compound I (50 mg/kg i.p.)** | 0/10 | 0 | NS | -100% |
| **Compound I (100 mg/kg i.p.)** | 0/6 | 0 | NS | -100% |
| **Compound I (200 mg/kg i.p.)** | 0/6 | 0 | NS | -100% |

| | | | | |
|---|---|---|---|---|
| NS = Not Significant; * = p < 0.05; ** = p < 0.01; *** = p < 0.001. | | | | |

### Conclusions

Valproate displayed anticonvulsant activity in this model therefore the experiment was considered valid. The results show the presence of anticonvulsant activity for Compound I over the dose-range 20 - 200 mg/kg, i.p. in the audiogenic seizure test in DBA/2 mouse.

Neither valproate nor Compound I caused any significant changes in the number of deaths, providing evidence for the safety of Compound I.

### Example 5: Evaluation of Novel Resorcinol for Anticonvulsant Activity using the mouse 6 Hz Psychomotor Test

The 6Hz Psychomotor Test, which detects anticonvulsant activity, follows that described by Brown et al. (J. Pharmacol. Exp. Ther. 107, 273-283, 1953).

### Methods

### Study Details:

Male RjOrl: Swiss mice, 5 weeks old, weighing 28 - 38 g at the beginning of the experiment. Animals were acclimatized to the test facility for at least 5 days after delivery and randomly housed in groups of 3-4 in macrolon cages on wood litter with free access to food and water.

The animal house was maintained under artificial lighting (12 hours) between 7:00 and 19:00 in a controlled ambient temperature of 22 ± 2°C, and relative humidity between 30-70%.

Before transcorneal stimulation, a drop of tetracaine solution (2%) was applied on each eye of the mouse for local anesthesia. Between 1-10 minutes later, at the pretreatment time specified for the test compound, the mice were administered a rectangular current (44 mA, rectangular pulse: 0.2 ms pulse width, 3 s duration, 6 Hz) via corneal electrodes connected to a constant current shock generator.

The number of seizures as reflected by forelimb clonus were recorded immediately after current administration. Forelimb clonus was scored as absent (0 = no forelimb clonus), mild (1 = clonus with one forelimb) and strong (2 = clonus with both forelimbs).

15 mice were studied per group. The experimenters were blinded to the treatment. The cages were randomly assigned to a treatment code, each designating one treatment group.

Compound I was administered intraperitoneally at 20 and 50 mg/kg, 30 minutes before the test, and at 100 and 200 mg/kg, 60 minutes before the test and were compared with the vehicle control (5% ethanol, 10% Kolliphor EL, 85% physiological saline).

Valproate (300 mg/kg i.p.), administered 30 minutes before the test, was used as reference substance and was compared with the vehicle control (5% ethanol, 10% Kolliphor HS15, 85% physiological saline).

**Table 8: Treatment schedule**

| **Group** | **Number of animals** | **Treatment (route of administration, pretreatment time)** | **Dose-level (mg/kg)** | **Concentration (mg/mL)** | **Administration Volume (mL/kg)** |
|---|---|---|---|---|---|
| **1** | 15 males | Vehicle (i.p., -30 min) | 0 | 0 | 10 |
| **2** | 15 males | Valproate (i.p., -30 min) | 300 | 30 | 10 |
| **13** | 15 males | Compound I (i.p., -30 min) | 20 | 2 | 10 |
| **14** | 15 males | Compound I (i.p., -30 min) | 50 | 5 | 10 |
| **15** | 15 males | Compound I (i.p., -60 min) | 100 | 10 | 10 |
| **16** | 15 males | Compound I (i.p., -60 min) | 200 | 20 | 10 |

### Test Compounds:

Test compound: Compound I dissolved in 5% ethanol, 10% Kolliphor HS15, 85% physiological saline.

Vehicle control: 5% ethanol, 10% Kolliphor HS15, 85% physiological saline.

Reference: Valproate (300 mg/kg), dissolved in 5% ethanol, 10% Kolliphor HS15, 85% physiological saline.

### Statistical analysis:

Data were assumed to be non-normally distributed based on validation data, therefore non-parametric tests were utilized. Quantitative data (scores) with the test substance were analyzed by comparing treated groups with the corresponding vehicle control using Kruskal-Wallis test with Dunn's multiple comparisons test when the Kruskal-Wallis Test was significant. Quantitative data with the reference substance was analysed using Mann-Whitney U test.

### Results

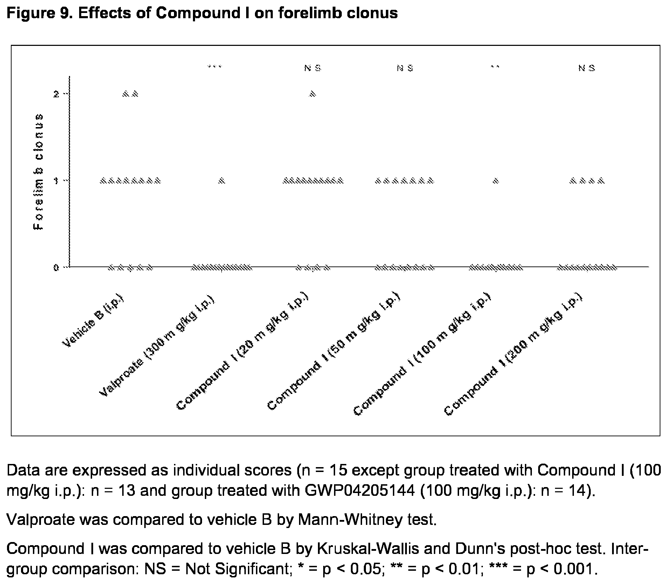

Figure 9 and Table 9 describe the data produced in this experiment.

As for the negative control, in vehicle controls administered i.p. 30 minutes before the test, 5 mice had a forelimb seizure score of 0, 8 mice had a forelimb seizure score of 1 and 2 mice had a forelimb seizure score of 2 (see Figure 9, furthest left).

As for the positive control, valproate (300 mg/kg), administered i.p. 30 minutes before the test, significantly decreased the mean forelimb seizure score, as compared with vehicle control (-88%, p < 0.001) (Figure 9, second left). One mouse out of the 15 tested showed slight sedation.

Two mice in the Compound I 100 mg/kg group and 1 mouse in the 200 mg/kg group were mis-dosed therefore excluded. Two mice out of the 14 in the Compound I 200 mg/kg group tested showed tremor and all mice showed slight (12 mice) to moderate (2 mice) sedation. Two mice out of the 13 in the Compound I 100 mg/kg dose group tested showed slight sedation.

There was a statistically significant difference in the Kruskal-Wallis test of forelimb seizure score between all Compound I dose groups as compared to vehicle (p < 0.01) (see Table 9).

Compound I (100 mg/kg), administered i.p. 60 minutes before the test, significantly decreased forelimb seizure score, as compared with vehicle control (-88%, p < 0.01) (Figure 9).

**Table 9: Effects of Compound I on forelimb clonus**

| **Treatment** | **Numberof mice per group** | **Forelimb clonus** | | | |
|---|---|---|---|---|---|
| | | **(mean ± s.e.m.)** | **Compared with Vehicle B (i.p.)** | | |
| | | | **(1)** | **(2)** | **%** |
| **Vehicle B (i.p.)** | 15 | 0.8 ± 0.2 | | | |
| **Compound I (20 mg/kg i.p.)** | 15 | 0.8 ± 0.1 | | NS | 0% |
| **Compound I (50 mg/kg i.p.)** | 15 | 0.5 ± 0.1 | ** | NS | -38% |
| **Compound I (100 mg/kg i.p.)** | 13 | 0.1 ± 0.1 | | ** | -88% |
| **Compound I (200 mg/kg i.p.)** | 14 | 0.3 ± 0.1 | | NS | -63% |
| **Valproate (300 mg/kg i.p.)** | 15 | 0.1 ± 0.1 | *** | | -88% |

| | | | | | |
|---|---|---|---|---|---|
| NS = Not Significant; * = p < 0.05; ** = p < 0.01; *** = p < 0.001. (1): For test substance-treated groups: Kruskal-Wallis Test. For reference-treated group: Mann-Whitney U test. (2): Dunn's multiple comparisons test when Kruskal-Wallis Test is significant. | | | | | |

### Conclusions

The results suggest the presence of anticonvulsant effects for Compound I at 100 mg/kg i.p. with a similar trend at 200 mg/kg in the 6 Hz (44mA) Psychomotor Seizure Test in the mouse.

### REFERENCES

A number of publications are cited above in order to more fully describe and disclose the invention and the state of the art to which the invention pertains. Full citations for these references are provided below.

Durairaj 2005. "Resorcinol Structure and Physical Properties." Resorcinol. Springer, Berlin, Heidelberg. https://doi.org/10.1007/3-540-28090-1_1

## Claims

1. A compound of formula (I), or a salt or a stereoisomer thereof:

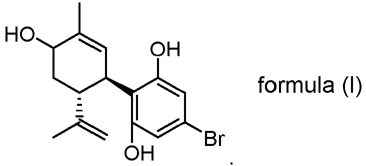

2. The compound of formula (I) of claim 1, wherein the compound is (1'R,2'R,4'S)-4-bromo-5'-methyl-2'-(prop-1-en-2-yl)-1',2',3',4'-tetrahydro-[1,1'-biphenyl]-2,4',6-triol.

3. A pharmaceutical composition comprising the compound of claim 1 or claim 2 together with one or more additional ingredients selected from carriers, diluents, excipients, adjuvants, fillers, buffers, binders, disintegrants, preservatives, antioxidants, lubricants, stabilisers, solubilisers, surfactants (e.g., wetting agents), masking agents, colouring agents, flavouring agents, and sweetening agents.

4. The pharmaceutical composition of claim 3, wherein the pharmaceutical composition is in a form selected from a liquid, a solution, a suspension, an emulsion, a syrup, an electuary, a mouthwash, a drop, a tablet, a granule, a powder, a lozenge, a pastille, a capsule, a cachet, a pill, an ampoule, a bolus, a suppository, a pessary, a tincture, a gel, a paste, an ointment, a cream, a lotion, an oil, a foam, a spray, and an aerosol.

5. The compound of claim 1 or claim 2 for use as a medicament.

6. The compound for use of claim 5, wherein the medicament is a medicament for treating epilepsy.

7. The compound for use of claim 5 or claim 6, wherein the medicament is a medicament for treating generalised seizure, focal onset seizure or tonic-clonic seizure.

8. The compound of claim 1 or claim 2 for use in a method of treatment.

9. The compound for use of claim 8, wherein the method of treatment is a method of treating epilepsy.

10. The compound for use of claim 8 or claim 9, wherein the method of treatment is a method of treating generalised seizure, focal onset seizure or tonic-clonic seizure.

11. A process for the production of a compound of formula (I) comprising the following steps:
i) Reacting 5-bromobenzene-1,3-diol with 4-isopropenyl-1-methyl-cyclohex-2-en-1-ol;
ii) Treating the resulting compound 5-Bromo-2-[6-isopropenyl-3-methyl-cyclohex-2-en-1-yl]benzene-1,3-diol with acetic anhydride;
iii) Treating the further resulting compound [3-Acetoxy-5-bromo-2-[6-isopropenyl-3-methyl-cyclohex-2-en-1-yl]phenyl] acetate with manganese acetate dihydrate;
iv) Adding sodium borohydride to the subsequently further resulting compound [3-Acetoxy-5-bromo-2-[(1*R*,6*R*)-6-isopropenyl-3-methyl-4-oxo-cyclohex-2-en-1-yl]phenyl] acetate, followed by subsequent steps to produce the compound of formula (I).

12. An intermediate formed in the process of the production of a compound of formula (I), wherein the intermediate is:

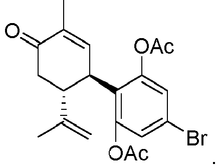

## Patentansprüche

1. Verbindung der Formel (I) oder Salz oder Stereoisomer davon:

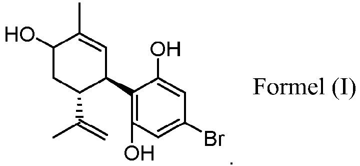

2. Verbindung der Formel (I) nach Anspruch 1, wobei die Verbindung (1'R,2'R,4'S)-4-Brom-5'-methyl-2'-(prop-1-en-2-yl)-1',2',3',4'-tetrahydro-[1,1'-biphenyl]-2,4',6-triol ist.

3. Pharmazeutische Zusammensetzung, umfassend die Verbindung nach Anspruch 1 oder Anspruch 2 zusammen mit einem oder mehreren zusätzlichen Inhaltsstoffen, die aus Folgenden ausgewählt sind: Trägern, Verdünnungsmitteln, Hilfsstoffen, Adjuvantien, Füllstoffen, Puffern, Bindemitteln, Zerfallsmitteln, Konservierungsmitteln, Antioxidantien, Schmiermitteln, Stabilisatoren, Lösungsvermittlern, Tensiden (z. B. Benetzungsmitteln), Maskierungsmitteln, Farbstoffen, Aromastoffen und Süßungsmitteln.

4. Pharmazeutische Zusammensetzung nach Anspruch 3, wobei die pharmazeutische Zusammensetzung in einer Form vorliegt, die aus Folgendem ausgewählt ist: einer Flüssigkeit, einer Lösung, einer Suspension, einer Emulsion, einem Sirup, einer Latwerge, einem Mundwasser, einem Tropfen, einer Tablette, einem Granulat, einem Pulver, einer Lutschtablette, einer Pastille, einer Kapsel, einer Oblatenkapsel, einer Pille, einer Ampulle, einem Bolus, einem Suppositorium, einem Pessar, einer Tinktur, einem Gel, einer Paste, einer Salbe, einer Creme, einer Lotion, einem Öl, einem Schaum, einem Spray und einem Aerosol.

5. Verbindung nach Anspruch 1 oder Anspruch 2 zur Verwendung als ein Medikament.

6. Verbindung zur Verwendung nach Anspruch 5, wobei das Medikament ein Medikament zum Behandeln von Epilepsie ist.

7. Verbindung zur Verwendung nach Anspruch 5 oder Anspruch 6, wobei das Medikament ein Medikament zum Behandeln eines generalisierten Anfalls, eines fokalen Anfalls oder eines tonisch-klonischen Anfalls ist.

8. Verbindung nach Anspruch 1 oder Anspruch 2 zur Verwendung in einem Behandlungsverfahren.

9. Verbindung zur Verwendung nach Anspruch 8, wobei das Behandlungsverfahren ein Verfahren zum Behandeln von Epilepsie ist.

10. Verbindung zur Verwendung nach Anspruch 8 oder Anspruch 9, wobei das Behandlungsverfahren ein Verfahren zum Behandeln eines generalisierten Anfalls, eines fokalen Anfalls oder eines tonisch-klonischen Anfalls ist.

11. Verfahren zur Produktion einer Verbindung der Formel (I), umfassend die folgenden Schritte:
i) Reagierenlassen von 5-Brombenzol-1,3-diol mit 4-Isopropenyl-1-methyl-cyclohex-2-en-1-ol;
ii) Behandeln der resultierenden Verbindung 5-Brom-2-[6-isopropenyl-3-methyl-cyclohex-2-en-1-yl]benzol-1,3-diol mit Essigsäureanhydrid;
iii) Behandeln der weiteren resultierenden Verbindung [3-Acetoxy-5-brom-2-[6-isopropenyl-3-methyl-cyclohex-2-en-1-yl] phenyl]acetat mit Manganacetatdihydrat;
iv) Hinzufügen von Natriumborhydrid zu der anschließend weiteren resultierenden Verbindung [3-Acetoxy-5-brom-2-[(1*R*,6*R*)-6-isopropenyl-3-methyl-4-oxo-cyclohex-2-en-1-yl]phenyl]acetat, gefolgt von nachfolgenden Schritten, um die Verbindung der Formel (I) zu produzieren.

12. Zwischenprodukt, das bei dem Prozess zur Produktion einer Verbindung der Formel (I) gebildet wird, wobei das Zwischenprodukt Folgendes ist:

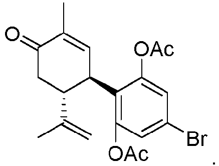

## Revendications

1. Composé de formule (I), ou sel ou stéréoisomère de celui-ci :

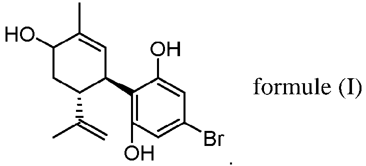

2. Composé de formule (I) de la revendication 1, dans lequel le composé est le (1'R,2'R,4'S)-4-bromo-5'-méthyl-2'-(prop-1-én-2-yl)-1',2',3',4'-tétrahydro-[1,1'-biphényl]-2,4',6-triol.

3. Composition pharmaceutique comprenant le composé de la revendication 1 ou la revendication 2 avec un ou plusieurs ingrédients supplémentaires choisis parmi les supports, les diluants, les excipients, les adjuvants, les charges, les tampons, les liants, les délitants, les conservateurs, les antioxydants, les lubrifiants, les stabilisants, les solubilisants, les tensioactifs (par exemple, les agents mouillants), les agents masquants, les agents colorants, les aromatisants et les édulcorants.

4. Composition pharmaceutique de la revendication 3, ladite composition pharmaceutique étant sous une forme choisie parmi un liquide, une solution, une suspension, une émulsion, un sirop, un électuaire, un bain de bouche, une goutte, un comprimé, un granulé, une poudre, une tablette, une pastille, une capsule, un cachet, une pilule, une ampoule, un bolus, un suppositoire, un pessaire, une teinture, un gel, une pâte, une pommade, une crème, une lotion, une huile, une mousse, un vaporisateur et un aérosol.

5. Composé de la revendication 1 ou la revendication 2 destiné à être utilisé en tant que médicament.

6. Composé destiné à être utilisé de la revendication 5, ledit médicament étant un médicament destiné à traiter l'épilepsie.

7. Composé destiné à être utilisé de la revendication 5 ou la revendication 6, ledit médicament étant un médicament destiné au traitement d'une crise généralisée, d'une crise focale ou d'une crise tonico-clonique.

8. Composé de la revendication 1 ou la revendication 2, destiné à être utilisé dans un procédé de traitement.

9. Composé destiné à être utilisé de la revendication 8, ledit procédé de traitement étant un procédé de traitement de l'épilepsie.

10. Composé destiné à être utilisé de la revendication 8 ou la revendication 9, ledit procédé de traitement étant un procédé de traitement de crise généralisée, de crise focale ou de crise tonico-clonique.

11. Processus permettant la production d'un composé de formule (I) comprenant les étapes suivantes :
i) la réaction du 5-bromobenzène-1,3-diol avec le 4-isopropényl-1-méthyl-cyclohex-2-én-1-ol ;
ii) le traitement du composé résultant, le 5-bromo-2-[6-isopropényl-3-méthyl-cyclohex-2-én-1-yl]benzène-1,3-diol, avec de l'anhydride acétique ;
iii) le traitement du composé résultant supplémentaire, l'acétate de [3-acétoxy-5-bromo-2-[6-isopropényl-3-méthyl-cyclohex-2-én-1-yl]phényle], avec de l'acétate de manganèse dihydraté ;
iv) l'ajout du borohydrure de sodium au composé résultant obtenu ultérieurement, l'acétate de [3-acétoxy-5-bromo-2-[(1*R*,6*R*)-6-isopropényl- 3-méthyl-4-oxo-cyclohex-2-én-1-yl]phényle], suivi d'étapes ultérieures pour produire le composé de formule (I).

12. Intermédiaire formé dans le processus de production d'un composé de formule (I), ledit intermédiaire étant :
